(19) Europäisches Patentamt
European Patent Office
Office européen des brevets

(11) Numéro de publication: **0 425 362 A2**

# (12) DEMANDE DE BREVET EUROPEEN

(21) Numéro de dépôt: **90402972.5**

(22) Date de dépôt: **23.10.90**

(51) Int. Cl.5: **C07D 307/52**, A61K 31/34

(30) Priorité: **24.10.89 IT 2211889**

(43) Date de publication de la demande: **02.05.91 Bulletin 91/18**

(84) Etats contractants désignés: **AT BE CH DE DK ES FR GB GR IT LI LU NL SE**

(71) Demandeur: **ROUSSEL-UCLAF**
**35, boulevard des Invalides**
**F-75007 Paris(FR)**

(72) Inventeur: **Pellegata, Renato L.**
**Via Cenisio 18**
**Milan(IT)**

(74) Mandataire: **Niel, Michel et al**
**ROUSSEL-UCLAF Boîte postale no 9 111, route de Noisy**
**F-93230 Romainville(FR)**

## (54) Sels de furosémide, procédé de préparation, application comme médicaments et compositions pharmaceutiques les renfermant.

(57) L'invention concerne de nouveaux sels de furosémide, en particulier des sels d'acides aminés basiques d'origine naturelle, sont utiles pour la préparation de compositions pharmaceutiques sous forme solide, liquide ou spray pour le traitement de sujets asthmatiques, le procédé pour la préparation desdits sels, et les compositions pharmaceutiques les renfermant.

EP 0 425 362 A2

## SELS DE FUROSEMIDE, PROCÉDÉ DE PRÉPARATION, APPLICATION COMME MÉDICAMENTS ET COMPOSITIONS PHARMACEUTIQUES LES RENFERMANT.

La présente invention concerne de nouveaux sels de furosémide, leur procédé de préparation, leur application comme médicaments et les compositions pharmaceutiques les renfermant.

Le furosémide ou l'acide 4-chloro N-furyl 5-sulfamoyl anthranylique est un composé très utilisé dans la pratique médicale comme agent diurétique, antihypertensif et antihypercalcémique.

On a récemment découvert que le furosémide, administré par aérosol à des sujets asthmatiques atopiques, est aussi en mesure de prévenir le bronchospasme induit par les exercices physiques, les brouillards ultrasoniques et les stimulations immunologiques.

Au cours de ces études, des patients ont aspiré, pendant 15 à 20 minutes, un aérosol obtenu par nébulisation d'une solution aqueuse contenant 10 mg/ml de furosémide, sel sodique, formulation commercialisée pour l'administration parentérale ; cette formulation présente une concentration proche du point de saturation.

Ce type d'administration est long et compliqué et, de ce fait, une telle utilisation du produit pharmaceutique dans la pratique thérapeutique courante est quasiment impossible.

Dans le brevet GB-B 2 189 239, publié le 1er février 1989, on décrit des sels de furosémide avec des bases d'ammonium quaternaire qui sont beaucoup plus solubles et plus biodisponibles que le sel de sodium correspondant. Toutefois, ces sels ne sont pas aptes à l'administration par aérosol, étant donné que les bases d'ammonium quaternaire et leurs sels provoquent très souvent un bronchoconstriction paroxystique chez le patient.

On a maintenant découvert que des sels de furosémide avec des acides aminés basiques, notamment ceux d'origine naturelle, tels que l'ornithine, l'arginine et la lysine peuvent être utilisés pour la préparation de compositions pharmaceutiques qui présentent de nombreux avantages par rapport à celles connues actuellement.

Par exemple, elles sont plus stables du point de vue chimique et physique, offrent une meilleure biodisponibilité du furosémide et permettent la préparation de formulations spray avec lesquelles on peut administrer, par un petit jet, des quantités de furosémide équivalentes à celles que l'on peut administrer par aérosol en utilisant une solution aqueuse du sel de sodium.

Les sels de furosémide obtenus selon la présente invention ont une solubilité dans l'eau beaucoup plus élevée que le furosémide lui-même ou son sel de sodium. Par exemple, le sel de furosémide avec la L-lysine présente une solubilité dans l'eau d'environ 560 mg/ml, ce qui correspond à une concentration de furosémide d'environ 400 mg/ml, et une solubilité dans une solution physiologique d'environ 490 mg/ml, ce qui correspond à une concentration en furosémide d'environ 310 mg/ml.

La présente invention a donc pour objet les sels de formule générale (I) :

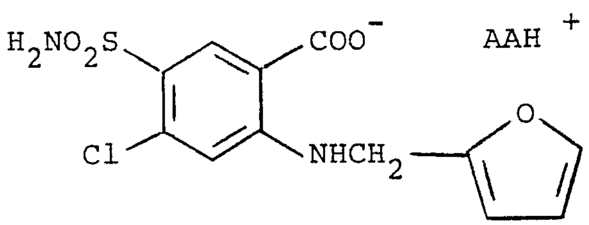

(I)

dans laquelle AA représente un acide aminé basique, tel que la lysine, l'arginine ou l'ornithine sous forme optiquement active ou racémique.

L'invention a notamment pour objet les composés de formule (I) dans laquelle l'acide aminé basique est la L-lysine ou la DL-lysine.

La présente invention a également pour objet un procédé de préparation de sels de furosémide solubles dans l'eau, ledit procédé consistant à faire réagir le furosémide lui-même avec un des acides aminés basiques au sein d'un solvant ou d'un mélange de solvants approprié, tel que l'eau, le méthanol, l'éthanol, à une température comprise entre 0 °C et la température d'ébullition du solvant ou des solvants utilisés.

Le sel peut ainsi être par précipitation dans le milieu de réaction ou isolé par d'autres méthodes usuelles connues en chimie organique. Le produit brut peut être purifié si nécessaire par recristallisation dans un solvant ou mélange de solvants approprié.

Les composés de formule (I) présentent des propriétés pharmacologiques intéressantes notamment dans le traitement de l'asthme et l'invention a donc pour objet les composés de formule (I) telle que définie ci-dessus à titre de médicaments.

De plus, les nouveaux sels de formule (I) qui renferment, comme contre-ion, un des acides aminés naturels peuvent être utilisés comme médicaments aptes à être administrés à des sujets hypertendus et qui ne présentent pas le risque de provoquer une réaction de bronchoconstriction paroxystique quand il est administré par aérosol (contrairement aux sels d'ammonium quaternaires).

La posologie varie en fonction du sujet traité et de la voie d'administration ; elle peut varier par exemple de 20 mg à 200 mg par jour chez l'adulte.

Les nouveaux composés de formule (I) tels que définis ci-dessus peuvent être employés pour préparer des compositions pharmaceutiques renfermant à titre de principe actif, l'un au moins desdits produits.

L'invention a donc pour objet les compositions pharmaceutiques renfermant comme agent thérapeutiquement actif, une quantité pharmacologiquement efficace d'au moins un composé de formule (I) telle que définie ci-dessus et un véhicule et/ou excipient pharmacologiquement acceptable.

Ces compositions pharmaceutiques peuvent être, par exemple, solides ou liquides et se présenter sous les formes pharmaceutiques couramment utilisées en médecine humaine comme par exemple les comprimés simples et dragéifiés, les gélules, les granulés, les solutions, les sprays, les aérosols, les préparations injectables. Elles sont préparées selon les méthodes usuelles. Le ou les principes actifs peuvent être incorporés à des excipients habituellement employés dans ces compositions pharmaceutiques, tels que le talc, le lactose, l'amidon, le stéarate de magnésium, les véhicules aqueux ou non, les glycols, les divers agents mouillants ou dispersants.

Comme les sels objet de la présente invention sont extrêmement solubles dans l'eau et que la biodisponibilité d'un médicament est proportionnelle à sa vitesse de solubilisation dans ce milieu, ces sels peuvent être utilisés pour la préparation de compositions pharmaceutiques sous forme solide dans lesquelles le furosémide présente une solubilité beaucoup plus élevée que les préparations pharmaceutiques solides commercialisées actuellement.

De plus, compte-tenu de leurs caractéristiques de stabilité, ces sels peuvent être utilisés aussi bien pour la préparation de compositions pharmaceutiques destinées à l'administration orale du médicament sous forme solide ou liquide que pour la préparation de compositions pharmaceutiques destinées à la voie parentérale ou inhalatrice.

Le tableau suivant contient des données de stabilité du furosémide sel de L-lysine en solution aqueuse.

TABLEAU

| Sol N° | Temp. de conserv. | Solvant | Conc. mg/ml | pH ① | Titre en furosémide ② après | | | | |
|---|---|---|---|---|---|---|---|---|---|
| | | | | | 10j | 26j | 37j | 7m | 12m |
| 1 | 25° C | eau | 97,2 | 7,00 | - | 97,7 | 96,8 | | |
| 2 | 25° C | sol. physiol | 184,4 | 7,09 | - | 99,6 | 98,7 | | |
| 3 | 25° C | eau | 275,3 | 7,32 | - | 99,6 | 98,2 | | |
| 4 | 60° C | eau | 125,0 | 7,06 | 99,3 | | | | |
| 5 | 25° C ③ | eau | 125,0 | 7,06 | 100,0 | | | | |
| 6 | 25° C | eau | 90,4 | 6,95 | 99,4 | - | 98,9 | | |
| 7 | 4° C | eau ④ | 72,1 | 7,06 | | | | 99,9 | 99,1 |

NOTES:

1) Mesure initiale du pH.
2) Exprimé en pourcentage par rapport au titre initial de furosémide.
3) Conservé à l'abri de la lumière.
4) Contenant 4% en poids de mannitol.

En particulier, en ce qui concerne la voie inhalatrice, les sels de furosémide, objet de la présente invention, permettent la préparation de formulations spray qui peuvent débiter en quelques petits jets (2 à 4) la quantité de furosémide thérapeutiquement active, en évitant les long délais (15 à 20 minutes) qui sont nécessaires actuellement et l'usage d'un pulvérisateur.

L'invention a plus particulièrement pour objet les compositions pharmaceutiques, sous forme de sprays, contenant un composé de formule (I) et un gaz propulsif tel que l'azote, l'air ou un autre gaz inerte.

En outre, on sait que l'administration d'un médicament par aérosol peut provoquer une brochoconstriotion paroxystique qui semble due à la composition de la solution aérosolisée. En particulier, celle-ci ne doit pas être hypotonique, ni contenir d'agents stabilisants, tels que l'EDTA, ni de conservateurs, tels que le métabisulfite ou les sels d'ammonium quaternaires, comme le chlorure de benzalconium. Or les sels de furosémide, objet de la présente invention, permettent de préparer aisément des solutions isotoniques ou hypertoniques stériles, et comme il s'agit de sels de furosémide avec un acide aminé basique naturel, l'administration par aérosol ne provoque aucune bronchoconstriction paroxystique, comme il est, par contre, théoriquement possible avec les composés décrits dans le brevet GB. B 2 189 239 où le furosémide, salifié avec une base d'ammonium quaternaire, a donc la structure des conservateurs auxquels on impute ce dangereux effet collatéral.

Dans les exemples qui suivent, la présente invention est illustrée d'une façon plus exhaustive.

**Exemple 1 : Préparation du furosémide, sel de L-lysine.**

On mélange 200 ml d'eau, 150 g (0,45 mol) de furosémide et 118 ml (0,45 mol) d'une solution aqueuse à 50% p/p de L-lysine. On agite 30 minutes environ, ajoute 1 l d'éthanol à 95% et obtient une solution limpide. On refroidit la solution à 8-10° C et, en poursuivant l'agitation, ajoute encore 1 l d'éthanol à 95%. Au cours de cette adjonction le produit commence à précipiter. On maintient l'agitation pendant 30 minutes puis filtre sous pression réduite. Le produit est lavé avec un peu d'alcool, puis séché à l'étuve sous pression réduite à 40° C. On obtient ainsi 210 g (97% du théorique) d'un produit blanc cristallisé. F = 214-216° C (déc).

$[alpha]_D$ = + 4,5° (c = 2% dans l'eau).

| I.R. (Nujol) : | | |
|---|---|---|
| Long. d'onde | Intensité | Assignation |
| 3380 | faible | Φ NH |
| 3300 - 2000 | élargie | Φ $NH_3^+$ |
| 1600, 1500, 1500 | forte | Δ $NH_3^+$, noyau phényle, Φ C = 0 (groupe $-COO^-$) |
| 1410 | moyenne | bande caractéristique |
| 1330, 1268 | forte | bandes caractéristiques |
| 1160 | forte | Φ $SO_2$ |
| 975, 940, 840, 750 | moyenne | bandes caractéristiques |

| RMN (DMSO) : | | | |
|---|---|---|---|
| N° protons | type | Multiplicité | Δ ppm |
| 1 | aromatique | singulet | 8,44 |
| 1 | aromatique | doublet (j = 3Hz) | 7,55 |
| 1 | aromatique | singulet | 6,76 |
| 2 | aromatique | absorp. complexe | 6,35 (centre) |
| 2 | $CH_2$-NH-CH | doublet* (j = 3Hz) | 4,41 |
| 1 | CH-N | absorp. complexe | 3,38 |
| 2 | CH-$CH_2$-N | absorp. complexe | 3,0-2,55 |
| 6 | $CH_2$-$CH_2$-$CH_2$-CH | absorp. complexe | 2,05-1,15 |

**Exemple 2 : Préparation du furosémide, sel de DL-lysine.**

En procédant comme à l'exemple 1, mais en employant la DL-lysine au lieu de la L-lysine, on obtient avec un rendement de 98%, le sel de furosémide correspondant, qui présente les caractéristiques suivantes. F = 228-230 ° C (décomp).

| I.R. (Nujol) : | | |
|---|---|---|
| Long. d'onde | Intensité | Assignation |
| 3380 | faible | Φ NH |
| 3300 - 2000 | élargie | Φ $NH_3$ + |
| 1600, 1500, 1500 | forte | Δ $NH_3$ +, noyau phényle, Φ C = 0 (groupe $-COO^-$) |
| 1410, 1330, 1268 | forte | bande caractéristique |
| 1160 | forte | Φ $SO_2$ |
| 975, 940, 840, 750, 685 | moyenne | bandes caractéristiques |

**Exemple 3 : Préparation du furosémide, sel de L-ornithine.**

En procédant comme à l'exemple 1, mais en employant la L-ornithine au lieu de la L-lysine, on obtient avec un rendement de 92% le sel de furosémide correspondant, qui présente les caractéristiques suivantes. F = 202-203 ° C (décomp.).
$[alpha]_D$ = + 4,3 (c = 1% dans l'eau).

**Exemple 4 : Préparation du furosémide, sel de DL-ornithine.**

En procédant comme à l'exemple 1, mais en employant la DL-ornithine au lieu de la L-lysine, on obtient avec un rendement de 94% le sel de furosémide correspondant, qui présente les caractéristiques suivantes. F = 188-189 ° C (décomp.).

**Exemple 5 : Préparation du furosémide, sel de L-arginine.**

En procédant comme à l'exemple 1, mais en employant la L-arginine au lieu de la L-lysine, on obtient avec un rendement de 82% le sel de furosémide correspondant, qui présente les caractéristiques suivantes. F = 180-183 ° C (décomp.).
$[alpha]_D$ = + 5,5 (c = 1% dans le mélange eau-éthanol 4/1).

**Exemple 6 : Préparation du furosémide, sel de DL-arginine.**

En procédant comme à l'exemple 1, mais en employant la DL-arginine au lieu de la L-lysine, on obtient avec un rendement de 84% le sel de furosémide correspondant, qui présente les caractéristiques suivantes. F = 186-190 ° C (décomp.).

**Exemple 7 : Composition et procédé pour la préparation d'une formulation spray de furosémide, sel de L-lysine.**

| A) Composition : | |
|---|---|
| Un litre de solution contient : | |
| - Furosémide, sel de lysine (correspondant à 50 g de furosémide) .... | 72,1 g |
| - Mannitol .... | 40 g |
| - Eau p.p.i. ....q.s.p. | l litre |

B) Procédé de préparation :

Dans 2 tiers de la quantité d'eau p.p.i. prévue, on dissout le mannitol puis le furosémide sel de L-lysine ; on ajoute alors le tiers restant de l'eau p.p.i. (le pH de la solution résultante varie entre 6,9 et 7,1). Cette solution est filtrée sur membrane stérilisante (0,22 µm) puis répartie en milieu stérile, dans des ampoules de 5 ml dépyrogénées, sous surpression d'azote très pur. Un petit jet de la susdite formulation débite environ 0,1 ml de solution (correspondant à 7,22 mg du sel de L-lysine du furosémide, soit à 5 mg de furosémide).

**Exemple 8 : Composition et procédé pour la préparation d'une formulation (forme sèche) de furosémide, sel de L-lysine destinée à l'inhalation.**

| A) Composition : | |
|---|---|
| Un kilogramme de mélange contient : | |
| - Furosémide, sel de lysine (correspondant à 250 g de furosémide) .... | 361 g |
| - Mannitol .... | 639 g |

B) Procédé de préparation :

Les deux composés sont intimement mélangés puis micronisés afin d'obtenir une taille de particules comprise entre 5 et 10 microns. La poudre est ensuite répartie en gélules de 40 mg (correspondant à 14,44 mg du sel de L-lysine du furosémide soit 10 mg de furosémide).

Le contenu de la gélule peut être administré au moyen d'un inhalateur à poudre couramment utilisé en pratique médicale.

**Revendications**

1.- Composés de formule (I) :

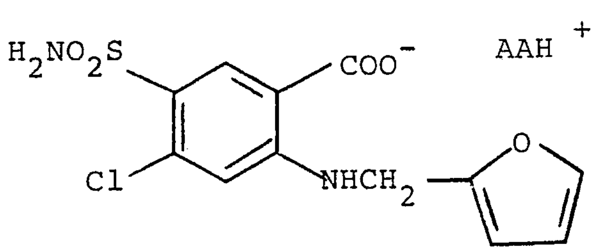

(I)

dans laquelle AA représente un acide aminé basique, tel que la lysine, l'arginine ou l'ornithine sous forme optiquement active ou racémique.

2.- Composé de formule (I) selon la revendication 1, caractérisé en ce que l'acide aminé basique AA est la

L-lysine.
3.- Composé de formule (I) selon la revendication 1, caractérisé en ce que l'acide aminé basique AA est la DL-lysine.
4. - Procédé pour la préparation des composés de formule (I), caractérisé en ce que l'on fait réagir le furosémide avec un acide aminé basique (AA), tel que la lysine, l'arginine ou l'ornithine, au sein d'un solvant ou d'un mélange de solvants approprié à une température comprise entre 0° C et la température d'ébullition du solvant ou des solvants utilisés.
5. - A titre de médicaments, les composés de formule (I) telle que définie à l'une quelconque des revendications 1 à 3.
6.- Composition pharmaceutique, caractérisée en ce qu'elle contient comme agent thérapeutiquement actif, une quantité pharmacologiquement efficace d'au moins un composé de formule (I) selon la revendication 1 et un véhicule et/ou excipient pharmacologiquement acceptable.
7. - Composition pharmaceutique selon la revendication 6 sous forme de spray, caractérisée en ce qu'elle contient un compose de formule (I) et un gaz propulsif tel que l'azote, l'air ou un autre gaz inerte.

Revendications pour l'Etat contractant suivant : ES

1.- Procédé pour préparer les composés de formule (I) :

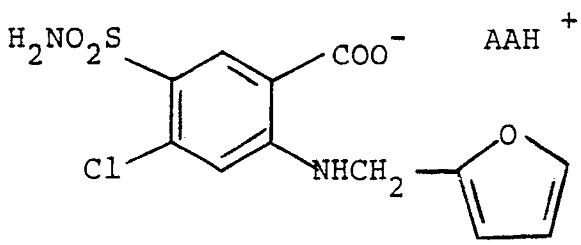

(I)

dans laquelle AA représente un acide aminé basique, tel que la lysine, l'arginine ou l'ornithine sous forme optiquement active ou racémique, caractérisé en ce que l'on fait réagir le furosémide avec un acide aminé basique (AA), tel que la lysine, l'arginine ou l'ornithine, au sein d'un solvant ou d'un mélange de solvants approprié à une température comprise entre 0° C et la température d'ébullition du solvant ou des solvants utilisés.
2.- Procédé selon la revendication 1, caractérisé en ce que l'on utilise comme acide aminé basique AA est la L-lysine.
3.- Procédé selon la revendication 1, caractérisé en ce que l'on utilise comme acide aminé basique AA est la DL-lysine.

Revendications pour l'Etat contractant suivant : GR

1.- Procédé pour préparer les composés de formule (I) :

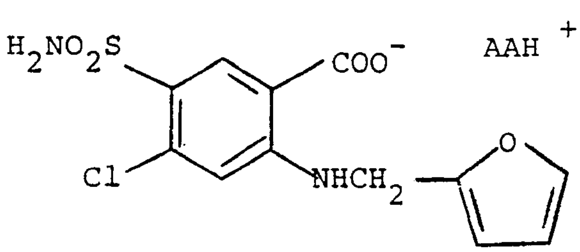

(I)

dans laquelle AA représente un acide aminé basique, tel que la lysine, l'arginine ou l'ornithine sous forme optiquement active ou racémique, caractérisé en ce que l'on fait réagir le furosémide avec un acide aminé basique (AA), tel que la lysine, l'arginine ou l'ornithine, au sein d'un solvant ou d'un mélange de solvants approprié à une température comprise entre 0° C et la température d'ébullition du solvant ou des solvants utilisés.
2.- Procédé selon la revendication 1, caractérisé en ce que l'on utilise comme acide aminé basique AA est la L-lysine.

3.- Procédé selon la revendication 1, caractérisé en ce que l'on utilise comme acide aminé basique AA est la DL-lysine.

4.- Procédé de préparation de compositions pharmaceutiques, caractérisé en ce que l'on met à titre de principe actif l'un au moins des composés de formule générale (I) telle que définie à l'une quelconque des revendications 1 à 3, pharmaceutiquement acceptables sous une forme destinée à cet usage.

5. - Procédé de préparation de compositions pharmaceutiques, caractérisé en ce que l'on met à titre de principe actif l'un au moins des composés de formule générale (I) telle que définie à l'une quelconque des revendications 1 à 3, pharmaceutiquement acceptables sous forme de spray, caractérisée en ce qu'elle contient un composé de formule (I) et un gaz propulsif tel que l'azote, l'air ou un autre gaz inerte.